# EUROPEAN PATENT APPLICATION

(11) **EP 2 377 862 A1**
(43) Date of publication of application: **19.10.2011**
(21) Application number: 10382070.0
(22) Date of filing: 29.03.2010
(51) Int. Cl.: C07D 411/04

(54) **Process for obtaining emtricitabine**

(71) Applicant: Esteve Química, S.A., 08024 Barcelona (ES)
(72) Inventor: Bartra Sanmarti, Marti, E 08024, BARCELONA (ES); Berenguer Maimo, Ramón, E-08024, BARCELONA (ES); Solsona Rocabert, Joan Gabriel, E 08024 BARCELONA (ES)
(74) Representative: Arias Sanz, Juan

(57) **Abstract**

The invention refers to a process for obtaining or isolating emtricitabine which comprises the azeotropic distillation of methanol-B(OMe)₃. The invention is also directed to emtricitabine having a content of boron species of not more than 0.1 % by weight.

## Description

### FIELD OF THE INVENTION

The present invention relates to a process for obtaining emtricitabine. More particularly, the invention is directed to a process for isolating emtricitabine in a high degree of purity.

### BACKGROUND

Emtricitabine (FTC) is a nucleoside reverse transcriptase inhibitor (NRTI) for the treatment of HIV having the following formula (I)

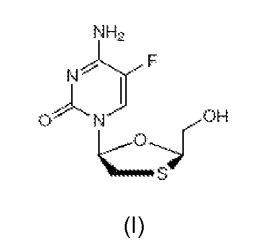

This antiviral agent, having 4-amino-5-fluoro-1-[(2*R*,5*S*)-2-(hydroxymethyl)-1,3-oxathiolan-5-yl]-2(1*H*)-pyrimidinone as chemical name and [143491-57-0] as CAS number, is also known under the trade name Emtriva (formerly Coviracil) (Merck Index, Thirteenth Ed., 2001, No. 3597).

Emtricitabine has two chiral centers and therefore four stereoisomers exist, namely (2R, 5S) and (2S, 5R) which are the cis isomers and (2R, 5R) and (2S, 5S) which are the trans isomers. The cis (2R, 5S) isomer is found to have profound antiviral activity while the other cis (2S, 5R) isomer and the trans isomers (2S, 5S) and (2R, 5R) have lower therapeutic activity and are found to be of reduced interest in therapeutics.

The synthetic approach adopted for emtricitabine involves introduction of the pyrimidine base in the 1,3-oxathiolane ring. There are various processes used in the prior art for the preparation of emtricitabine using different synthetic approaches.

For example, the preparation of the racemic mixture of cis isomers via standard reactions followed by enzymatic resolution to give the desired (2R, 5S) enantiomer is disclosed in WO 92/14743. The resolution can also be achieved by HPLC chiral, such as passing the enantiomers of cis-emtricitabine through an acetylated β-cyclodextrin chiral column.

Several stereoselective processes have been subsequently developed, which mainly by means of chiral auxiliaries such as menthol, allow the desired stereochemistry to be induced. Typically, the reductive removal of the chiral auxiliary affords emtricitabine as single enantiomer in these routes, as illustrated in the scheme below reflecting the process from L-menthyl emtricitabine [(2R,5S)-((1 R,2S,5R)-2-isopropyl-5-methylcyclohexyl) 5-(4-amino-5-fluoro-2-oxopyrimidin-1 (2H)-yl)-1,3-oxathiolane-2-carboxylate]:

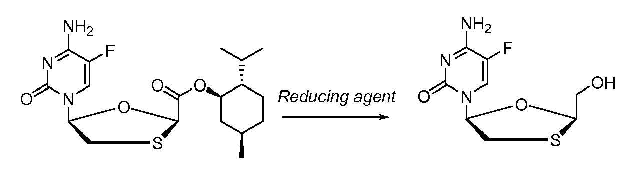

US 5,696,254 discloses a process for preparing emtricitabine, which involves condensation of L-menthyl cis-1,3-oxathiolan-5S-acetoxy-2R-carboxylate with 5-fluorocytosine in the presence of iodotrimethylsilane and reduction of the L-menthyl ester group of the product so formed with lithium aluminum hydride to afford emtricitabine (see example 21 therein). In the example 2 of US 5,538,975 the reduction is also carried out with the same hydride. However, lithium aluminum hydride is a pyrophoric material which reacts strongly in contact with water or humid air, and thus these processes require special handled conditions. Further, a silica gel column chromatography is also needed. These drawbacks prevent its industrial application. On the other hand, US 5,696,254 shows discrepancies relating to the stereochemistry of the intermediates used to obtain emtricitabine, since examples 18 and 19 relates to intermediate compounds unsuitable for producing emtricitabine.

RED-Al (sodium bis(2-methoxyethoxy)aluminumhydride), other reducing agent sensitive to humidity, is employed in the reduction of L-menthyl emtricitabine disclosed in CN1274687C, which further encompasses a column chromatography.

Boron hydrides are more easily handled as they are much more stable to humidity. However, the use of these reducing agents also presents inconveniences or disadvantages such as additional steps or tedious purifications to remove the boron side species formed during the reduction. Further, in the present case, this aspect is especially significant due to the high solubility of emtricitabine and the boron species in water.

In CN10166971A, the reduction with KBH₄ does not afford emtricitabine directly but its hydroxybenzoic salt, thus involving the additional step of obtaining emtricitabine in free base form.

Several references describe the use of NaBH₄ as reducing agent of L-menthyl emtricitabine (see for instance EP 1 608 644, WO 2007/077505, CN101407513A, WO 2009/084033). However, these processes show important drawbacks as well. For instance, in WO 2009/084033 the reaction gives place to the hydrochloride salt, and therefore an additional step is carried out to obtain the ingredient active in the free base form. Likewise, with the exception of CN101407513A, the chemical yield reported when using NaBH₄ is low (up to 57%). Further, some of these procedures require several solvents or additives in the reaction step and also evaporations to dryness. Furthermore, as noted above, it is believed that emtricitabine obtained according to said procedures presents certain amounts of boron species as well as inorganic salts. In this regard, it is worth mentioning that the inventors of the instant invention when reproducing the example 4 of CN101407513A have found that it does not afford the final product free of inorganic salts and boron species, since said salts precipitate under the crystallization conditions.

In view of the above, there does not exist any example in the state of the art which allows to prepare emtricitabine in good yield and high purity by means of a readily industrializable process.

It is therefore necessary to solve the problems associated with the processes belonging to the state of the art and provide an easy cost-effective process for obtaining emtricitabine which improves its isolation and thus being advantageously applicable at industrial scale. In particular, said improved process should afford the product with good yield and quality avoiding the need of any further purification and additionally it should take place in absence of additives.

### BRIEF DESCRIPTION OF THE INVENTION

The invention faces the problem of providing an improved process for obtaining emtricitabine, which overcomes all or part of the problems existing in the different aforementioned syntheses of the state of the art. In particular, as the use of boron reducing agents is found to be advantageous in its synthesis, a solution for obtaining this ingredient active substantially free of boron impurities and/or inorganic salts would be highly desirable.

The solution provided by the present invention is based on the fact that the inventors have observed that it is surprisingly possible to isolate substantially pure emtricitabine from a crude reaction mixture containing side boron species by means of azeotropic distillation of methanol-B(OMe)₃.

More particularly, said boron species in presence of metanol give place to B(OMe)₃ which is removed by means of azeotropic distillation of methanol-B(OMe)₃.

Furthermore, said boron species in presence of an alcohol give place to a borate ester of formula (lV), which, by adding MeOH, is subsequently transformed into B(OMe)₃ (III), which is removed by means of azeotropic distillation of methanol-B(OMe)₃. A distillation allows the equilibrium of the transesterification reaction to shift to the right due to the high volatility of the azeotrope formed, as may be observed in the following scheme.

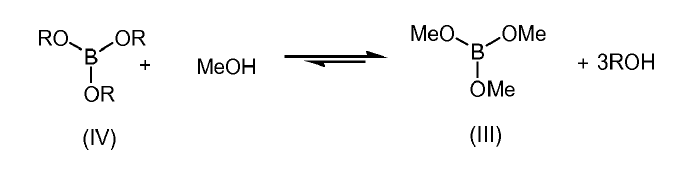

Therefore, one aspect of the present invention relates to a process, hereinafter process of the invention, for obtaining or isolating emtricitabine which comprises:
a) mixing methanol with a mixture comprising emtricitabine and boron species; and
b) performing an azeotropic distillation of methanol-B(OMe)₃.

In a particular embodiment, the present invention relates to a process for obtaining or isolating emtricitabine which comprises:
a) mixing at least a C₂₋₈ alkyl alcohol with a first mixture comprising emtricitabine and boron species to form a second mixture comprising emtricitabine and a borate ester;
b) mixing methanol with the mixture obtained in step a); and
c) performing an azeotropic distillation of methanol-B(OMe)₃.

Preferably, the process of the invention above may additionally comprise a filtration step in order to remove insoluble salts. Such filtration may be performed in any stage.

Additionally, it is preferred that the mixture comprising emtricitabine and boron species is anhydrous, before performing the azeotropic distillation of methanol-B(OMe)₃.

In the context of the invention, emtricitabine may be prepared according to any of the processes disclosed in the state of the art. However, as the skilled person will appreciate, the process for isolating emtricitabine provided herein is particularly advantageous when it has been obtained through a synthetic pathway comprising at least one step wherein a boron reagent is used. For example, but not limited to, the stereoselective processes wherein the chiral auxiliary is removed by a reductive reaction with a boron reducing agent.

Therefore, a preferred embodiment of the present invention relates to a process for obtaining emtricitabine which comprises:
a) the reduction of an intermediate compound of formula (II) 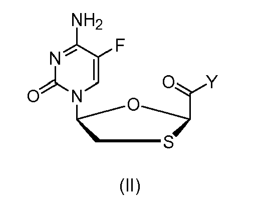 wherein Y represents a chiral auxiliary,
   with a boron reducing agent to obtain a mixture comprising emtricitabine and boron species;
b) optionally, mixing at least a C₂₋₈ alkyl alcohol with the mixture obtained in step a);
c) mixing methanol with the mixture obtained in the previous step; and
d) performing an azeotropic distillation of methanol-B(OMe)₃.

In an especially preferred embodiment, the boron reducing agent is NaBH₄.

Another aspect of the present invention relates to substantially pure emtricitabine, particularly emtricitabine having a content of boron species, expressed as H₃B0₃, of not more than a 0.5% by weight, preferably of not more than 0.1% by weight; more preferably said emtricitabine has a content of inorganic salts of not more than 0.1% by weight.

In another aspect, the invention is directed to emtricitabine obtainable according to the process of the invention.

Also provided according to the invention is a pharmaceutical composition comprising emtricitabine having a minimum content of boron species. It is also provided by the present invention a pharmaceutical composition comprising emtricitabine having a minimum content of boron species and of inorganic salts as defined above together to a pharmaceutically acceptable carrier, adjuvant or vehicle.

These aspects and preferred embodiments thereof are additionally also defined in the claims.

### DETAILED DESCRIPTION OF THE INVENTION

In the context of the present invention, the following terms have the meaning detailed below:
"C₂₋₈ Alkyl" refers to a straight or branched hydrocarbon chain radical consisting of carbon and hydrogen atoms, containing no unsaturation, having two to eight carbon atoms, and which is attached to the rest of the molecule by a single bond, e. g., ethyl, n-propyl, i-propyl, n-butyl, t-butyl, n-pentyl, etc.

Emtricitabine can be obtained in free form or, alternatively, in the form of salt. In both cases it can be obtained in crystalline form, both as free compounds or as solvates (e.g., hydrates, alcoholates, etc.), both forms being included within the scope of the present invention. The solvation methods are generally known in the state of the art.

As a whole, a process such as the one disclosed in the present invention provides emtricitabine in good yield and pharmaceutical quality and at the same time avoids any further purification step. Likewise, said process allows using non-hazardous reactants. All of this contributes to reducing the overall cost of the process, making it commercially interesting and allowing it to be put into practice on an industrial level.

As noted previously, the process for isolating emtricitabine may be applied to any mixture of this active ingredient containing boron species. However, it is preferably carried out after a reductive step with a boron reducing agent. According to the process of the invention, the intermediate compound of formula (II) may be subjected to a reduction reaction with a boron reducing agent which generates hydride ions, under conditions allowing the reduction of the group in position 2 to a hydroxyl group, to obtain the compound of formula (I) (emtricitabine). 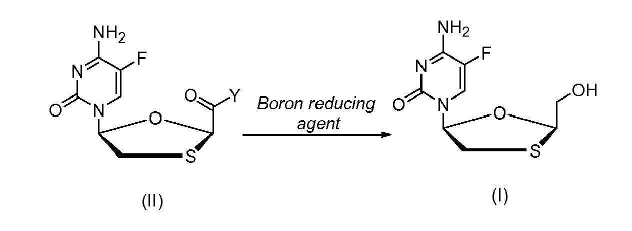 wherein Y represents a chiral auxiliary.

Illustrative, non-limiting examples of suitable conditions to carry out the reaction of reduction are for instance those disclosed in EP 1608644 (example 1), WO 2007/077505 (example 4), CN101407513A (examples 4-6), WO 2009/084033 (example 2). The isolation of emtricitabine after the reduction is performed following the process of the invention.

The term "chiral auxiliary" describes asymmetric molecules that are used to effect the chemical resolution of a racemic mixture. Such chiral auxiliaries may possess one chiral center such as methylbenzylamine or several chiral centers such as menthol. The purpose of the chiral auxiliary, once built into the starting material, is to allow simple separation of the resulting diastereomeric mixture. See, for example, J. Jacques et al., Enantiomers, Racemates and Resolutions, pp. 251-369, John Wiley & Sons, New York (1981). Examples of chiral auxiliaries suitable in the present invention are for instance amines and alcohols such as methylbenzylamine and menthol, respectively. The L-menthol [(1 R,2S,5R)-5-methyl-2-propan-2-ylcyclohexan-1-ol] is preferred.

The optical purity of the compounds can be determined by chiral HPLC methods, specific rotation measurements and NMR techniques. As a general rule, if the opposite enantiomer is desired, it may be obtained by using the mirror image of the chiral auxiliary initially employed. In the present case, L-menthol is used to afford the (2R, 5S) isomer of emtricitabine and therefore, its enantiomer (2S, 5R) could be obtained by using D-menthol. The selection of the appropriate configuration for other chiral auxiliaries is a routine work for the skilled person.

As used herein, a "boron reducing agent" is a compound capable of transferring one or more hydride ions and which are formed or contain boron and one or more electropositive elements selected from groups IA (alkaline metals), IIA (alkaline earth metals) or IIIA, such as for example, lithium, sodium, potassium, aluminum, etc.; in a particular embodiment, the reducing agent which generates hydride ions has an anion (e.g., a cyanide group (CN-), etc.). Although virtually any boron reducing agent which generates hydride ions can be used to implement the process of the invention, in a particular embodiment said reducing agent is selected from sodium borohydride (NaBH₄), potassium borohydride (KBH₄), lithium borohydride (LiBH₄), sodium cyanoborohydride (NaBH₃CN), diborane (B₂H₆), or lithium triethylborohydride (LiEt₃BH or lithium super-hydride), and their mixtures. The diborane can be a diborane generated in the medium by means of, for example, l₂/sodium borohydride (NaBH₄), BF₃0Et₂/NaBH₄, by means of reacting NaBH₄ with BF₃ adducts with di-n-butylether, tert-butylether, monoglyme, dioxane or tetrahydropyran (Inorg. Chem., 2000, 39(8):1795-1802), etc.;or a commercial diborane in solution such as, for example, BH₃/Me₂S, etc. Preferably, the boron reducing agent is NaBH₄.

According to a particular embodiment, the reduction of the intermediate compound (II) to emtricitabine may be carried out in a suitable solvent (or a mixture of solvents), such as an organic solvent, for example, an alcohol, such as a low molecular weight alcohol (e.g., methanol, ethanol, isopropanol, etc.), an ether, for example, an aliphatic ether (e.g., diisopropyl ether, etc.), a cyclic ether (e.g., tetrahydrofuran (THF), methyl-THF, a dioxane, etc.), an halogenated solvent (e.g., dichloromethane, 1,2 dichloroethane, etc.), an aromatic solvent (e.g., toluene, xylene, etc.), etc.

The reduction reaction is suitably carried out under cooling, for example at a temperature comprised between - 75 °C and 10 °C, for a time period equal to or greater than 1 hour, typically comprised between 1 and 24 hours, allowing the reduction to the hydroxyl group. The development of the reaction can be monitored by different means such as TLC. Once the reaction is completed, the removal of the free chiral auxiliary can be achieved employing different common techniques such as extraction.

In a preferred embodiment, emtricitabine is obtained from L-menthyl emtricitabine by means of reduction using NaBH₄ as reducing agent and methanol as solvent, at a reaction temperature comprised between -10°C and 10°C. For instance, at -5°C the reaction is completed in about 4.5 or 5 hours. The menthol released is preferably removed by extraction with toluene.

A mixture containing emtricitabine and boron species, such as a crude reaction mixture obtained from the reduction of menthyl emtricitabine with a boron reducing agent, requires to be purified in order to obtain pharmaceutically acceptable emtricitabine.

The high solubility of emtricitabine and of the boron species in water complicates its separation.

As noted previously, it is convenient that the mixture of emtricitabine and boron species does not contain substantially water in order to achieve a good subsequent purification. Accordingly, if the mixture contains water, it may be dried by adding a solvent (e.g. isopropanol) which forms an azeotrope with water and carrying out a distillation of the azeotrope. Such distillation, for instance by means of an evaporation under reduced pressure, is more efficient if it is repeated twice or more times. Other suitable dehydrating methods include the addition of molecular sieves to the mixture. Before being subjected to the azeotropic distillation of methanol-B(OMe)₃, the water content in the mixture is preferably no more than 3% by weight, more preferably no more than 1% by weight, and even more preferably no more than 0.5% by weight.

According to a first embodiment of the process of the invention, the mixture containing emtricitabine and boron species, preferably anhydrous, is mixed with methanol to form B(OMe)₃. This reaction with methanol proceeds quickly.

According to a second embodiment of the process of the invention, the mixture containing emtricitabine and boron species, preferably anhydrous, is firstly mixed with at least one alcohol of formula general ROH, wherein R may independently represent any organic group provided that its borate (IV) is less volatile than the azeotrope MeOH-B(OMe)₃ (III)- In this regard, as the boiling point increases with increasing molecular mass, any alcohol can be employed since the MeOH-B(OMe)₃ azeotrope has the lowest boiling point. By way of example, the following table shows that the borate esters resulting from alcohols similar to methanol (EtOH, iPrOH, nPrOH) have a boiling point very high (respectively 120°C, 140 °C and 180°C) in comparison with B(OMe)₃ (69°C). The table shows also that the boiling point of the azeotrope MeOH-B(OMe)₃ (54°C) is lower than the azeotropes of EtOH-B(OEt)₃ (77°C) and ⁱPrOH-B(OⁱPr)₃ (82 °C). It is also to be noted that the azeotrope derived from methanol presents the highest boron content (73% wt. expressed as B(OMe)₃) and consequently it provides a better removal of the boron species.

| ROH | b.p.* ROH | B(OR)₃ | b.p*_{.} B(OR)₃ | azeotrope b.p.* ROH-B(OR)₃ | azeotrope B(OR)₃ % by weight |
|---|---|---|---|---|---|
| MeOH | 65 °C | B(OMe)₃ | 69 °C | 54 °C | 73% |
| EtOH | 78 °C | B(OEt)₃ | 120°C | 77 °C | 32% |
| ⁱPrOH | 82 °C | B(OⁱPr)₃ | 140 °C | 82 °C | 14% |
| ⁿPrOH | 97 °C | B(OⁿPr)₃ | 180 °C | --- | --- |

| | | | | | |
|---|---|---|---|---|---|
| ^{*} atmospheric pressure | | | | | |

From an experimental point of view, it is preferred that the alcohol is commercially available and at low cost. Therefore, preferably R is a C₂-₈ alkyl; most preferably, it is selected from ethanol, n-propanol and isopropanol. Nevertheless, the skilled person clearly understands that the mixture containing emtricitabine and boron species, may be also firstly mixed with at least one alcohol of formula general ROH, wherein R is preferably a C₂-₈ alkyl, and methanol.

The product of the reaction with the C₂-₈ alkyl alcohol is the borate ester of formula (IV) B(OR)₃. This reaction with the C₂-₈ alkyl alcohol proceeds quickly. Once the borate is formed, MeOH is added to the mixture, preferably anhydrous, to form B(OMe)₃.

Whether B(OMe)₃ is obtained from the mixture containing emtricitabine and boron species via the borate ester of formula (IV) or not (direct reaction of methanol), an azeotropic distillation is performed subsequently in order to remove the boron species. By this way, the side boron species can be completely separated from the emtricitabine and thus obtaining the active ingredient in substantially pure form. Afterwards, conventional crystallization techniques may be conveniently employed to isolate emtricitabine from the solution.

The present invention further provides pharmaceutical compositions comprising emtricitabine in substantially pure form as defined herein together with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

Emtricitabine or a composition thereof according to this invention may be used with other drugs to provide a combination therapy. The other drugs may form part of the same composition, or be provided as a separate composition for administration at the same time or at different time.

The features of the particular and preferred embodiments may be combined in the context of the present invention as long as the combination is not contradictory.

The following examples are merely illustrative of certain embodiments of the invention and cannot be considered as restricting it in any way.

### Examples

### Example 1. Preparation of emtricitabine via B(OⁱPr)₃

A suspension of (2R,5S)-((1 R,2S,5R)-2-isopropyl-5-methylcyclohexyl) 5-(4-amino-5-fluoro-2-oxopyrimidin-1(2H)-yl)-1,3-oxathiolane-2-carboxylate (30.0 g) in MeOH (300 mL) was cooled to -7 °C under a nitrogen atmosphere. Then, NaBH₄ (6.8 g) was added in portions for 1 h and the reaction mixture was stirred at -5 °C for 5 h. The resulting solution was heated to 5°C, water (105 mL) was added and was slowly titrated to pH 5.5 with 35% aqueous HCl solution at 10 °C. The solution was concentrated under reduced pressure to 140 mL total internal volume. Toluene (90 mL) was added and the layers were separated after 10 min stirring. The aqueous phase was washed again with toluene (2 x 90 mL) and, after phase separation, neutralized (pH 7.5) with 25% aqueous NaOH solution. Part of the solvent was evaporated under reduced pressure until reaching a internal volume of 60 mL. Celite (6 g) was charged to the aqueous solution and the mixture stirred for 5 min. Isopropanol (300 mL) was added and the solvent was evaporated under reduced pressure until the residual volume reached 75 mL. This last operation was repeated twice. Isopropanol (470 mL) was added and the suspension was heated to 60 °C, stirred for 1 h, filtered while hot and washed with hot isopropanol (2 x 30 mL). The filtrate and washing were combined and partially concentrated under reduced pressure to 75 mL total internal volume. MeOH (150 mL) was charged and the mixture was concentrated under reduced pressure to 75 mL, thus removing the boron species. Isopropanol (300 mL) was added and the solvent was evaporated under reduced pressure to 160 total internal volume. The suspension was heated to 80 °C and water (6 mL) was added. The resulting solution was cooled to 60 °C, seeded, stirred for 1 h at 60 °C, cooled to 0 °C and held at this temperature for 1 h. The solid was collected by filtration, washed with chilled isopropanol (2 x 15 mL) and dried in vacuo (40 °C) to give 12.1 g of emtricitabine (65% yield; purity by HPLC 99.77 area %; assay by HPLC 99.8% w/w; boric acid and chloride not detected by capillary electrophoresis).

### Example 2. Preparation of emtricitabine by direct addition of methanol

A suspension of (2R,5S)-((1 R,2S,5R)-2-isopropyl-5-methylcyclohexyl) 5-(4-amino-5-fluoro-2-oxopyrimidin-1 (2H)-yl)-1,3-oxathiolane-2-carboxylate (50.0 g) in MeOH (500 mL) was cooled to -7 °C under a nitrogen atmosphere. Then, NaBH₄ (10.2 g) was added in portions for 1.5 h and the reaction mixture was stirred at -5 °C for 4.5 h. The resulting solution was heated to 5°C and a 1.25 M HCl solution in methanol (220 mL) was slowly added. The mixture was concentrated under reduced pressure until the internal volume reached 250 mL, thus removing the boron species. Water (250 mL) was added and the pH was adjusted to 6. The solution was concentrated under reduced pressure to 250 mL total internal volume. Toluene (150 mL) was added and the layers were separated after 10 min stirring. The aqueous phase was washed again with toluene (2 x 150 mL) and, after phase separation, neutralized (pH 7.5) with 25% aqueous NaOH solution. Part of the solvent was evaporated under reduced pressure until reaching a internal volume of 100 mL. Celite (10 g) was charged to the aqueous solution and the mixture stirred for 5 min. Isopropanol (500 mL) was added and the solvent was evaporated under reduced pressure until the residual volume reached 125 mL. This last operation was repeated twice. Isopropanol (780 mL) was added and the suspension was heated to 60 °C, stirred for 1 h, filtered while hot and washed with hot isopropanol (2 x 50 mL). The filtrate and washing were combined and partially concentrated under reduced pressure to 265 mL total internal volume. The suspension was heated to 80 °C and water (10 mL) was added. The resulting solution was cooled to 60 °C, seeded, stirred for 1 h at 60 °C, cooled to 0 °C and held at this temperature for 1 h. The solid was collected by filtration, washed with chilled isopropanol (2 x 50 mL) and dried in vacuo (40 °C) to give 19.5 g of emtricitabine (63% yield; purity by HPLC 99.94 area %; assay by HPLC 99.7% w/w; boric acid and chloride not detected by capillary electrophoresis).

### Comparative Example: Preparation of emtricitabine by the example 4 of CN101407513 (compound l-B therein)

- The present inventors have found that the process disclosed in the example 4 of CN101407513A does not afford the final product free of inorganic salts and boron species, since said salts precipitate under the crystallization conditions. A solid was isolated providing the following results: Emtricitabine: assay by HPLC = 0.5%. Capillary electrophoresis analysis: chlorides 29%, boric acid 9%, unidentified intense peak (possibly phosphates). Emtricitabine was found to be in the mother liquors.
- Further, the process includes two steps which are hardly large-scale industrializable: i) pH correction of the organic phase after the reaction; ii) separation of toluene/water phases.

## Claims

1. A process for obtaining emtricitabine which comprises:
a) mixing methanol with a mixture comprising emtricitabine and boron species; and
b) performing an azeotropic distillation of methanol-B(OMe)₃.

2. A process according to claim 1 which comprises:
a) mixing at least a C₂₋₈ alcohol with a first mixture comprising emtricitabine and boron species to form a second mixture comprising emtricitabine and a borate ester;
b) mixing methanol with the mixture obtained in step a); and
c) performing an azeotropic distillation of methanol-B(OMe)₃.

3. A process according to any of claims 1 or 2, wherein the mixture comprising emtricitabine and boron species is anhydrous.

4. The process according to any of claims 2 or 3, wherein the C₂₋₈ alcohol is selected from ethanol, isopropanol and n-propanol.

5. The process according to any of claims 1 to 4 further comprising the previous step of reducing an intermediate compound of formula (II) 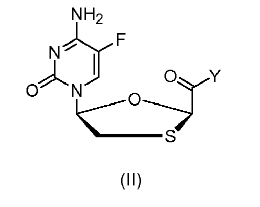 wherein Y represents a chiral auxiliary,
with a boron reducing agent to obtain a reaction mixture comprising emtricitabine and boron species.

6. The process according to claim 5, wherein the chiral auxiliary is L-menthol.

7. The process according to claim 5, wherein the boron reducing agent is selected from NaBH₄, KBH₄, LiBH₄, NaBH₃CN, B₂H₆ or LiEt₃BH and their mixtures.

8. The process according to claim 7, wherein the boron reducing agent is NaBH₄.

9. The process according to claim 5, wherein the reduction is carried out in a solvent comprising methanol.

10. Emtricitabine having a content of boron species, expressed as H₃BO₃, of not more than 0.1 % by weight.

11. Emtricitabine according to claim 10 having a content of inorganic salts of not more than 0.1 % by weight.

12. Emtricitabine obtainable according to the process defined in any of claims 1 to 9.

13. A pharmaceutical composition comprising emtricitabine as defined in any of claims 10, 11 or 12 and a pharmaceutically acceptable carrier, adjuvant or vehicle.
